(19) Europäisches Patentamt
European Patent Office
Office européen des brevets

(11) Numéro de publication : **0 021 927**
**B1**

(12) **FASCICULE DE BREVET EUROPÉEN**

(45) Date de publication du fascicule du brevet : **23.12.81**

(21) Numéro de dépôt : **80400811.8**

(22) Date de dépôt : **06.06.80**

(51) Int. Cl.³ : **C 07 C 41/16**, C 07 C 43/205, C 07 C 43/215, C 07 D303/22, C 07 C 43/23, C 07 C 67/31, C 07 C 69/712, C 07 C 93/04

# (54) Procédé de préparation d'éthers aryliques.

(30) Priorité : **20.06.79 FR 7915782**

(43) Date de publication de la demande : **07.01.81 (Bulletin 81/01)**

(45) Mention de la délivrance du brevet : **23.12.81 Bulletin 81/51**

(84) Etats contractants désignés : **AT BE CH DE FR GB IT LI LU NL SE**

(56) Documents cités : **Néant**

(73) Titulaire : **RHONE-POULENC INDUSTRIES**
**Brevets Pharma 25 Quai Paul Doumer**
**F-92408 Courbevoie Cedex (FR)**

(72) Inventeur : **Soula, Gérard**
**33, rue Nungesser**
**F-69330Meyzieu (FR)**
Inventeur : **Michelet, Daniel**
**24, Chemin de Montriblond**
**F-69160 Tassin-la-Demi-Lune (FR)**

(74) Mandataire : **Cazes, Jean-Marie et al**
**RHONE POULENC Service Brevets Chimie et Polymères B.P. 753**
**F-75360 Paris Cedex 08 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 17, rue du Louvre, 75001 Paris, France

EP 0 021 927 B1

# Procédé de préparation d'éthers aryliques

La présente invention a pour objet un procédé de préparation d'éthers aryliques. Elle concerne plus particulièrement la préparation d'éthers aryliques par réaction d'un halogénure aliphatique avec un phénolate ou un naphtolate.

Des procédés de ce type sont connus dans l'art antérieur. On connaît en particulier la synthèse d'éthers alkylaryliques par catalyse par transfert de phase c'est-à-dire par réaction d'un phénol en solution aqueuse avec un halogénoalcane en solution dans un solvant organique non miscible à l'eau en présence d'un ammonium quaternaire et d'une base minérale. Un procédé de ce type est décrit par exemple dans Synthesis 1973 p. 441-456 et dans Angew. Chem. Int. Ed. Engl. 13 p. 170-179 (1974).

L'inconvénient de ce type de procédé est qu'il nécessite des quantités importantes d'eau. Il est nécessaire, notamment, d'effectuer un traitement des eaux résiduaires avant leur rejet. D'autre part, il existe des cas particuliers où la présence d'eau est nuisibe : l'eau peut dégrader au moins en partie l'un des réactifs ; elle peut également favoriser des réactions secondaires comme par exemple des réactions de C-alkylation des polyphénols par des chloroalcényles.

On connaît aussi le brevet français 2 255 279 qui décrit la synthèse d'éthers alkylaryliques par réaction d'un halogénure aliphatique avec un phénol en présence d'un agent alcalin dans un solvant polaire.

Ce procédé bien que ne mettant pas en œuvre d'eau, n'est pas approprié pour une exploitation à l'échelle industrielle car il met en jeu des solvants chers et de manipulation malaisée.

La présente invention a pour but de pallier les inconvénients de l'art antérieur dans le cadre d'un procédé par catalyse par transfert de phase.

L'invention a donc pour objet un procédé de préparation d'éther arylique par réaction en milieu solvant organique d'un halogénure aliphatique avec un phénolate ou un naphtolate d'un métal alcalin ou alcalino-terreux ou d'ammonium, caractérisé en ce que la réaction a lieu en présence d'au moins un agent séquestrant de formule :

$$N{-}[CHR_1{-}CHR_2{-}O{-}(CHR_3{-}CHR_4{-}O)_n{-}R_5]_3 \qquad (I)$$

dans laquelle n est un nombre entier supérieur ou égal à 0 et inférieur ou égal à environ 10 ($0 \leq n \leq 10$), $R_1$, $R_2$, $R_3$ et $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical $-C_mH_{2m}-\emptyset$ ou $C_mH_{2m+1}-\emptyset-$, où m est compris entre 1 et 12.

L'intérêt de ce procédé résulte notamment du fait que le solvant mis en œuvre peut être un solvant apolaire, c'est-à-dire ne présentant pas à l'échelle industrielle les inconvénients des solvants utilisés jusqu'à présent. On peut même dans certains cas utiliser l'halogénure aliphatique lui-même comme solvant. Il est évident, bien que l'intérêt industriel, dans ce cas, soit nettement moins marqué, qu'il est également possible d'utiliser un solvant polaire.

L'invention repose sur le fait que l'agent séquestrant de formule I forme avec le phénolate ou le naphtolate un complexe et que ce complexe est soluble dans des solvants dans lesquels le phénolate ou le naphtolate est à l'état non complexé insoluble ou très peu soluble. Cette complexation a un double effet : en premier lieu, elle permet la solubilisation du phénolate ou naphtolate et de ce fait permet à la réaction d'avoir lieu ; en second lieu, bien que cela ne soit pas complètement expliqué, il semble qu'elle active le système réactionnel.

Selon un mode de réalisation préférentiel de l'invention, on utilise un agent séquestrant de formule (I) dans laquelle $R_1$, $R_2$, $R_3$ et $R_4$ représentent un atome d'hydrogène ou un radical méthyle, $R_5$ et n ayant la signification précédente.

Parmi ces derniers, on préfère encore plus particulièrement mettre en œuvre les agents séquestrants pour lesquels n est supérieur ou égal à 0 et inférieur ou égal à 6 et pour lesquels $R_5$ représente un radical alkyle ayant de 1 à 4 atomes de carbone.

On peut citer :

— la tris(oxa-3 butyl)amine de formule :

$$N{-}(CH_2{-}CH_2{-}O{-}CH_3)_3$$

— la tris(dioxa-3,6 heptyl)amine de formule :

$$N{-}(CH_2{-}CH_2{-}O{-}CH_2{-}CH_2{-}O{-}CH_3)_3$$

· ' tris(trioxa-3,6,9 décyl)amine de formule :

$$N{-}(CH_2{-}CH_2{-}O{-}CH_2{-}O{-}CH_2{-}CH_2{-}O{-}CH_3)_3$$

— la tris(dioxa-3,6 octyl)amine de formule :

$$N-(CH_2-CH_2-O-CH_2-CH_2-O-C_2H_5)_3$$

— la tris(trioxa-3,6,9 undécyl)amine de formule :

$$N-(CH_2-CH_2-O-CH_2-CH_2-O-CH_2-CH_2-O-C_2H_5)_3$$

— la tris(dioxa-3,6 nonyl)amine de formule :

$$N-(CH_2-CH_2-O-CH_2-CH_2-O-C_3H_7)_3$$

— la tris(trioxa-3,6,9 dodécyl)amine de formule :

$$N-(CH_2-CH_2-O-CH_2-CH_2-O-CH_2-CH_2-O-C_3H_7)_3$$

— la tris(dioxa-3,6 décyl)amine de formule :

$$N-(CH_2-CH_2-O-CH_2-CH_2-O-C_4H_9)_3$$

— la tris(trioxa-3,6,9 tridécyl)amine de formule :

$$N-(CH_2-CH_2-O-CH_2-CH_2-O-CH_2-CH_2-O-C_4H_9)_3$$

— la tris(tetraoxa-3,6,9,12 tridecyl)amine de formule :

$$N-[CH_2-CH_2-O-(CH_2-CH_2-O-)_3CH_3]_3$$

— la tris(hexaoxa-3,6,9,12,15,18 nonadecyl)amine de formule :

$$N-[CH_2-CH_2-O-(CH_2-CH_2-O-)_5-CH_3]_3$$

— la tris(dioxa-3,6 méthyl-4 heptyl)amine de formule :

$$N-[CH_2-CH_2-O-CH(CH_3)-CH_2-O-CH_3]_3$$

— la tris(dioxa-3,6 diméthyl-2,4 heptyl)amine de formule :

$$N-[CH_2-CH(CH_3)-O-CH(CH_3)-CH_2-O-CH_3]_3$$

Les amines utilisées dans le procédé selon l'invention sont connues en tant que telles dans l'art antérieur. C'est ainsi que le brevet français 1 302 365 cite l'obtention des amines tertiaires $N-(CH_2-CH_2-O-CH_3)_3$ et $N-(CH_2-CH_2-O-CH_2-CH_2-O-CH_3)_3$ comme sous-produits de la synthèse des amines primaires et secondaires correspondantes, ces amines primaires et secondaires étant des produits intéressants comme intermédiaires en vue de la synthèse de substances pharmaceutiques, comme inhibiteurs de corrosion comme intermédiaires en vue de la synthèse de produits chimiques intéressants en agriculture et comme émulsifiant.

Un autre avantage du procédé selon l'invention est que l'agent séquestrant utilisé est aisément recyclable soit par distillation, soit par extraction.

Les halogénures aliphatiques pouvant être mis en œuvre selon le procédé de l'invention ont pour formule générale :

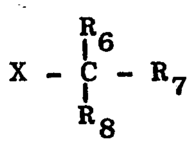

(II)

dans laquelle X représente Cl ou Br, $R_6$, $R_7$ et $R_8$ identiques ou différents représentent un élément choisi parmi le groupe comprenant l'hydrogène et les radicaux :

— alkyle ayant de 1 à 24 atomes de carbone,
— alcényle ayant de 2 à 24 atomes de carbone,
— alcynyle ayant de 2 à 24 atomes de carbone,
— phényle et naphtyle, éventuellement substitués,

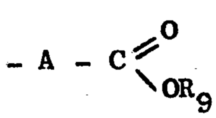

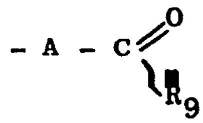

$-A-C\equiv N$

$-A-CR_9R_{10}OH$

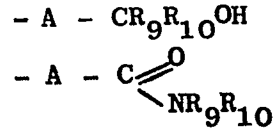

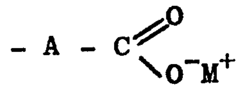

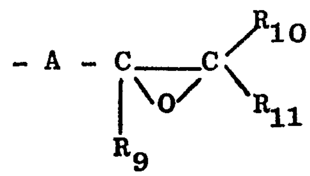

$-A-X$

où A représente un lien valentiel ou un reste hydrocarboné saturé ou insaturé, $R_9$, $R_{10}$ et $R_{11}$ identiques ou différents représentent un radical alkyle ayant de 1 à 12 atomes de carbone, un radical phényle ou l'hydrogène et où $M^+$ représente un cation alcalin ou alcalino-terreux, X ayant la signification précédente.

Les phénolates ou naphtolates pouvant être mis en œuvre dans le procédé selon l'invention ont pour formule :

$$Ar(O^-M^+)r \qquad (III)$$

dans laquelle : Ar représente un radical phényle ou naphtyle éventuellement substitué, $M^+$ représente un cation choisi parmi le groupe comprenant le cation ammonium, les cations dérivés des métaux alcalins et alcalino-terreux, et r est un nombre entier compris entre 1 et 3 ($1 \leq r \leq 3$).

Plus particulièrement, mais non exclusivement, l'invention concerne les composés de formules :

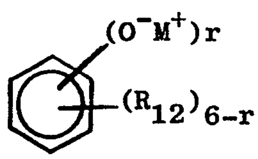

(IIIa) et

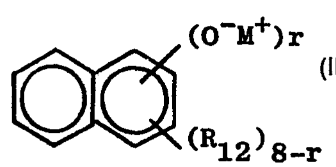

(IIIb)

dans lesquelles : r est égal à 1 ou 2, le ou les cations $M^+$, identiques ou différents, sont choisis parmi le groupe comprenant $Li^+$, $Na^+$, $K^+$, $NH_4^+$, $Ca^{2+}$, $Ba^{2+}$, le ou les radicaux $R_{12}$, identiques ou différents, sont choisis parmi le groupe comprenant :

— l'hydrogène

— les radicaux alkyle et cycloalkyle ayant de 1 à 12 atomes de carbone ;

— les radicaux alcényle ayant de 3 à 12 atomes de carbone comme les radicaux propényle, nonyle, dodécyle, par exemple ;

— le radical —OH

— les radicaux de formule $Cm\ H_{2m+1}\emptyset-$ ; $Cm\ H_{2m-1}\emptyset$ ; et $\emptyset-CmH_{2m}$ — où m est un nombre entier compris entre 1 et 12 ($1 \leq m \leq 12$) et où Ø peut être substitué

— les radicaux alkoxy ayant de 1 à 12 atomes de carbone et les radicaux phénoxy ;

— les radicaux $-CmH_{2m}-OH$ et $-CmH_{2m}OR_{13}$ où m est un nombre entier compris entre 1 et 12 ($1 \leq m \leq 12$) et où $R_{13}$ est un radical alkyle ayant de 1 à 12 atomes de carbone ou un radical phényle ;

— les radicaux alkylthio ayant de 1 à 12 atomes de carbone et les radicaux phénylthio ;
— les radicaux $CpH_{2p+1-q}$ Fq, p étant compris entre 1 et 4 ($1 \leq p \leq 4$) et q étant compris entre 3 et 9($3 \leq q \leq 9$) comme $-CF_3$ et $-CH_2-CF_3$ par exemple
— les radicaux

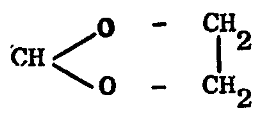

et

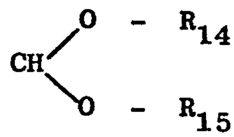

où $R_{14}$ et $R_{15}$ identiques ou différents représentent chacun un radical alkyle ayant de 1 à 12 atomes de carbone ou un radical phényle ;
— les radicaux Cl et F ;
— et les radicaux $-NO_2$, $NH_2$, $NHR_{14}$, $NR_{14}R_{15}$, $SO_3M$, CN, $-CO_2M$, $-CO_2R_{14}$, $-COR_{14}$, $-COH$, $-SO_2R_{14}$ où M représente un métal alcalin et où $R_{14}$ et $R_{15}$ ont la signification précédente.

On peut citer comme exemples d'halogénures aliphatiques de formule II les composés suivants : $CH_3-Cl$ ; $CH_3-Br$ ; $C_2H_5-Cl$ ; $C_2H_5Br$ ; $C_3H_7-Cl$ ; $CH_2=CH-CH_2-Cl$ ; $CH_2=C(CH_3)-CH_2Cl$ ; $CH_3-C(CH_3)_2-Cl$ ; $C_2H_5-C(CH_3)_2-Cl$ ; $C_3H_7-CH(CH_3)-Cl$ ; $nC_5H_{11}-Cl$ ; $nC_6H_{13}-Cl$ ; $C_3H_7-CH(C_2H_5)-Cl$ ; $nC_{10}H_{21}-Cl$ ; $nC_{10}H_{21}Br$ ; $CH\equiv C-CH_2-Cl$ ; $CH_3-C\equiv C-CH_2-CH_2-Cl$ ;

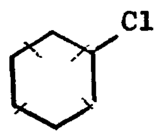

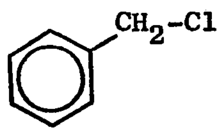

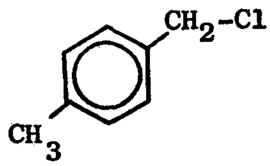

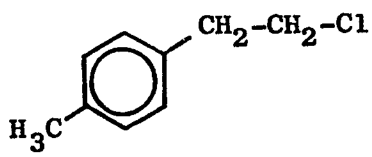

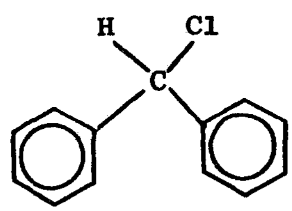

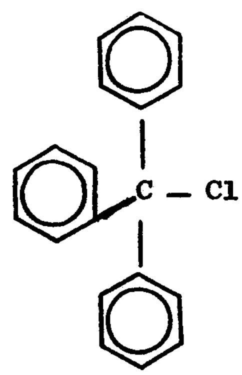

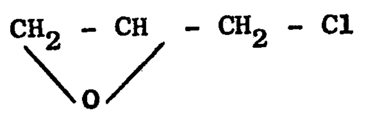 ; $Cl-CH_2-CO_2CH_3$ ; $Cl-CH_2-C\equiv N$

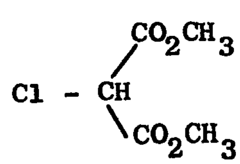 ; $Cl-CH_2-CO_2^-\ Na^+$ ; $Cl-CH_2-CH_2-CO_2^-Na^+$

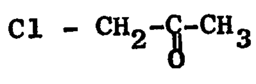 ; $Cl-CH(CH_3)-CO_2CH_3$ ;

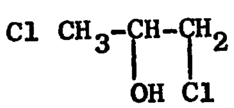 ; 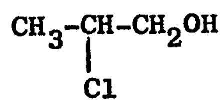 ; 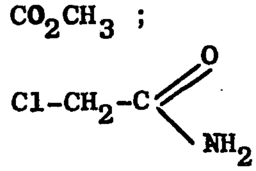

$Cl-CH_2-CH_2-Cl$ ; $Cl-CH_2-CH_2-CH_2-CH_2-Cl$

On peut citer comme exemples de phénolates et naphtolates de formule IIIa et IIIb, les composés

dérivés des phénols et naphtols suivants :

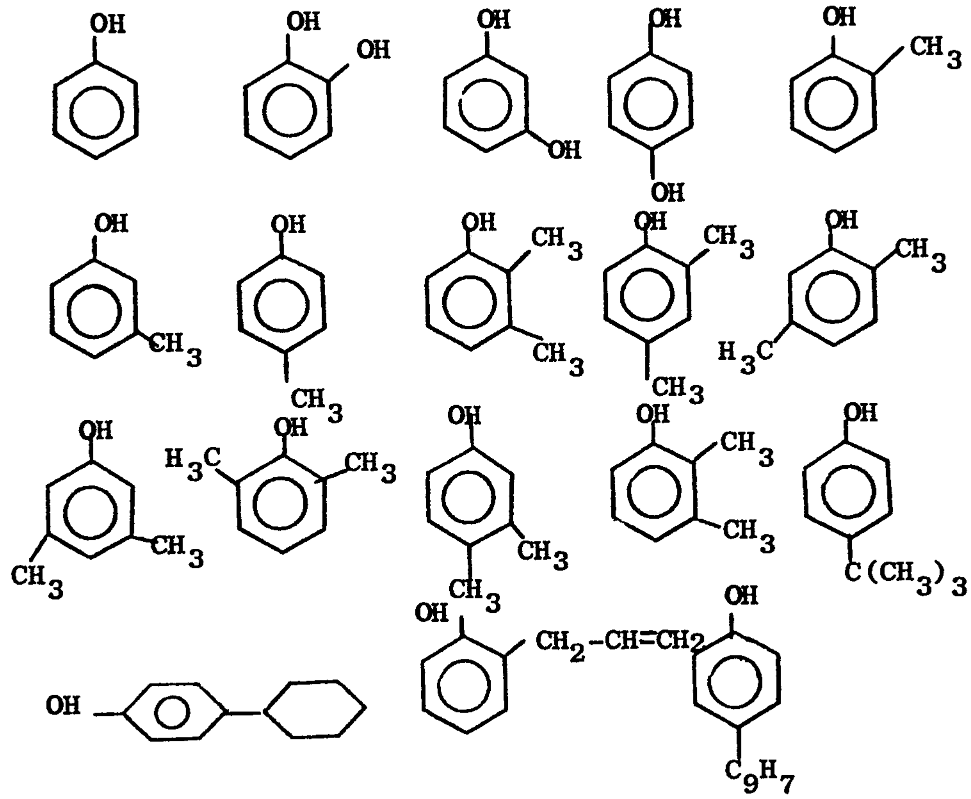

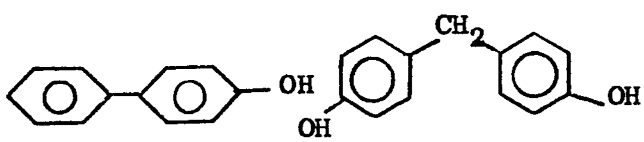

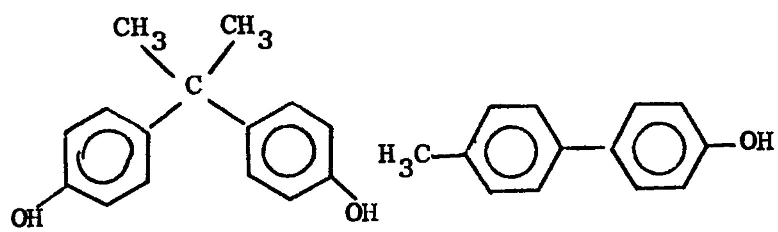

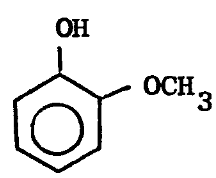

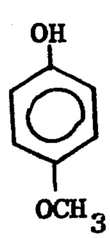

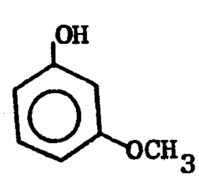

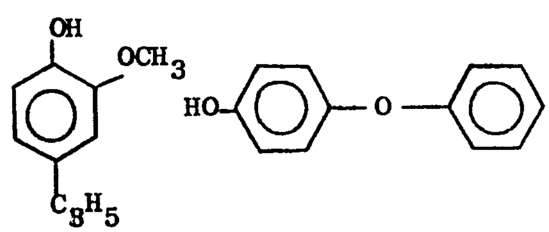

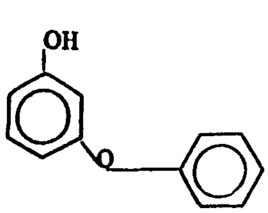 ; 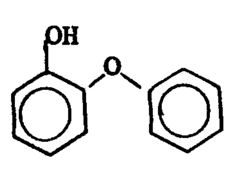 ; 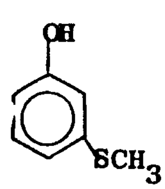 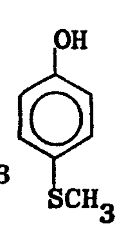 ;

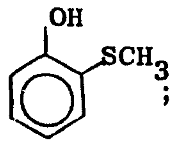 ; 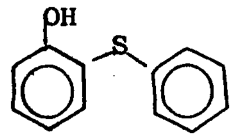 ; 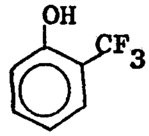 ; 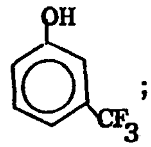 ; 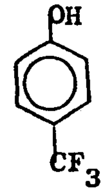 ;

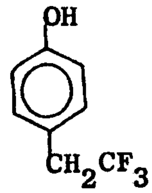 ; 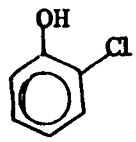 ; 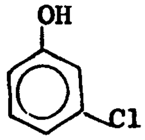 ; 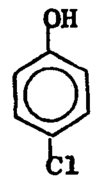 ; 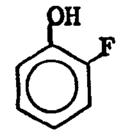 ; 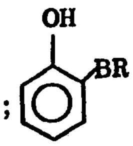

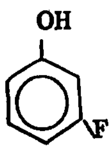 ; 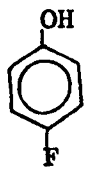 ; 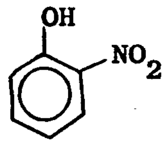 ; 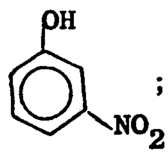 ; 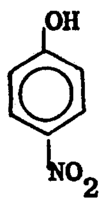 ; 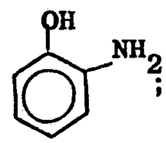 ;

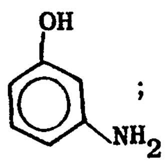 ; 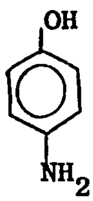 ; 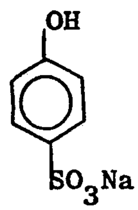 ; 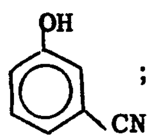 ; 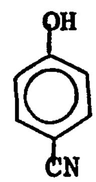 ; 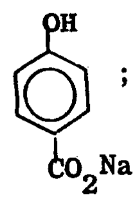 ;

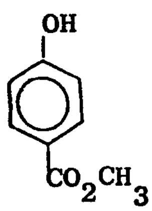 ; 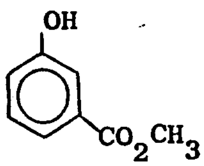 ; 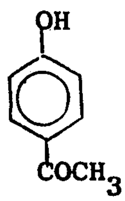 ; 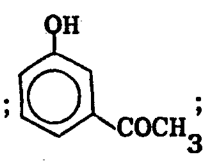 ; 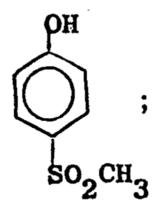 ;

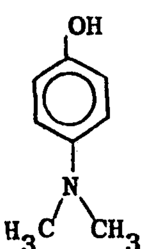 ; 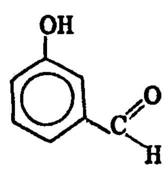 ; 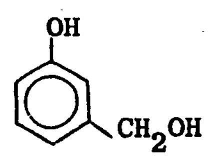 ; 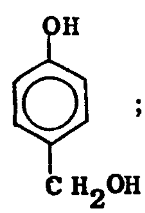 ;

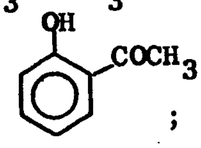 ; 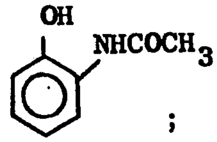 ; 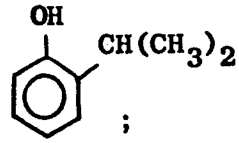 ;

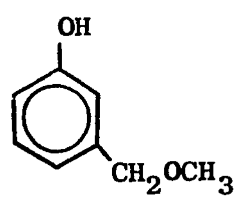 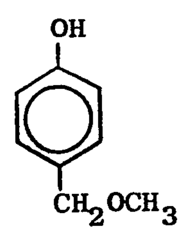 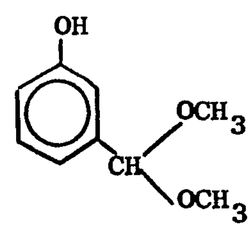 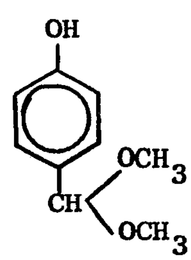

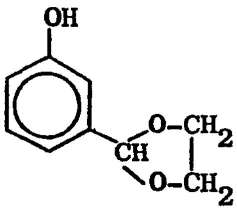 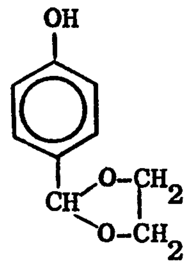 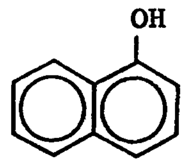 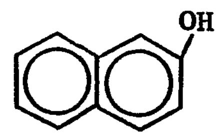

Le choix de l'agent séquestrant le plus adapté à la mise en œuvre du procédé selon l'invention doit être fait en tenant compte de la taille du cation $M^+$. Plus la taille du cation sera importante, plus le nombre d'atomes d'oxygène contenus dans la molécule de l'agent séquestrant devra être élevé. C'est ainsi que si on utilise un phénolate de potassium on préférera utiliser la tris(trioxa-3,6,9 décyl)amine alors qu'avec le sel de sodium correspondant, la tris(dioxa-3,6 heptyl)amine sera préférée.

Le solvant, lorsqu'on en utilise un, doit répondre à un certain nombre de conditions : il faut d'abord qu'il solubilise l'agent séquestrant (ce dernier est soluble dans la plupart des solvants usuels) ; il faut aussi qu'il soit inerte chimiquement vis-à-vis des sels à dissoudre. Il faut aussi noter que pour obtenir la meilleure mise en œuvre du procédé selon l'invention, plus le solvant choisi aura un caractère apolaire marqué, plus l'agent séquestrant devra avoir un caractère lipophyle marqué (c'est-à-dire plus l'agent séquestrant devra contenir d'atomes de carbone).

Les solvants utilisables dans le cadre du procédé selon l'invention sont par exemple : le benzène, le toluène, l'o, m et p-xylène, le monochlorobenzène, l'orthodichlorobenzène, le chloroforme, l'anisole, le diphényléther, l'acétonitrile, le dichloroéthane, le chlorobutane, le chlorure de benzyle, le nitrométhane, la diméthylformamide, la diméthylacétamide.

Le procédé selon l'invention doit être mis en œuvre à une température comprise entre environ − 25 °C et environ 150 °C.

On opère généralement à pression atmosphérique. Bien entendu, les pressions inférieures ou supérieures à la pression atmosphérique ne sont pas exclues par la présente invention.

On utilise l'agent séquestrant en quantité telle que le rapport molaire de l'agent séquestrant au phénolate ou au naphtolate est compris entre environ 0,5/100 et 15/100 de façon préférentielle ce rapport est compris entre 1/100 et 10/100.

Le rapport molaire de l'halogénure de formule II au phénolate ou naphtolate de formule III est compris entre environ 10 et environ 0,8. Les hautes valeurs de ce rapport correspondent au cas où l'halogénure est utilisé comme solvant.

Les composés obtenus selon le procédé de l'invention ont pour formule générale :

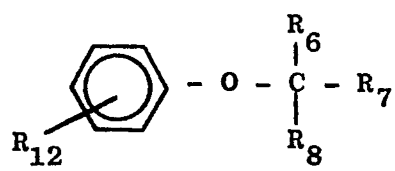 (IVa)

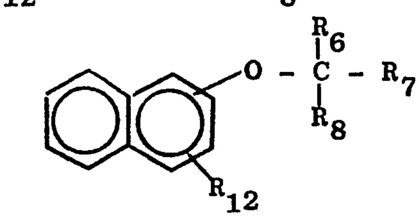 (IVb)

On peut citer comme exemples de composé IVa pour lequel le procédé selon l'invention est particulièrement bien adapté, les composés de formule :

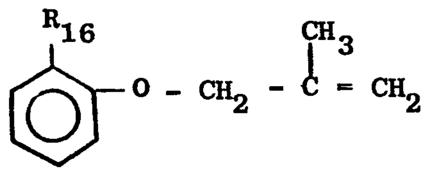

où $R_{16}$ représente un radical choisi parmi le groupe comprenant : OH, F, Cl, Br, $NO_2$ et les radicaux alkoxy, alcanoyle, (par exemple —CO—$CH_3$) ; —NHCO—alkyl et alkyl, ces radicaux ayant de préférence de 1 à 4 atomes de carbone, qui résulte de la réaction d'un sel alcalin du phénol :

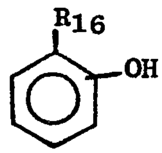

avec le chlorure de méthallyle.

D'autres exemples de composés IVa et IVb sont donnés ci-après :

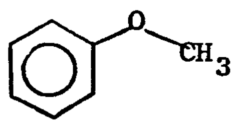

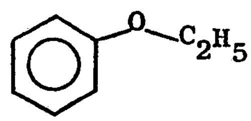

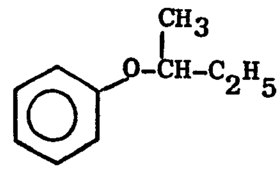

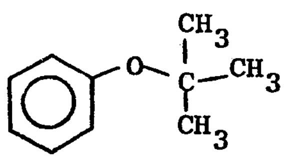

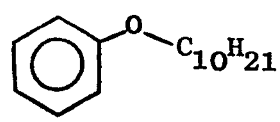

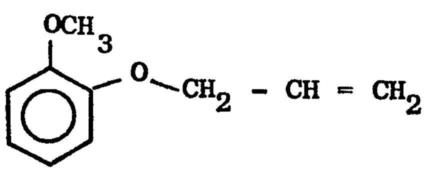

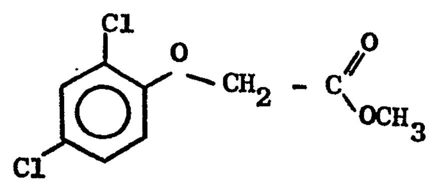

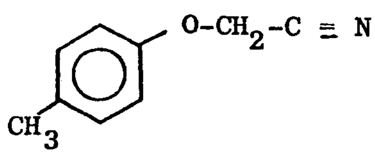

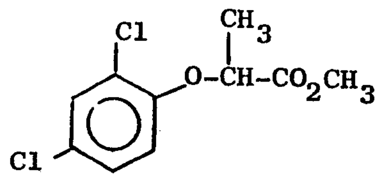

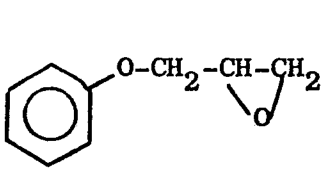 ; 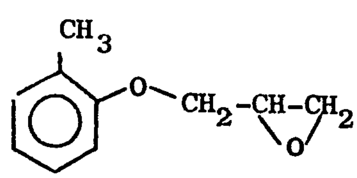 ;

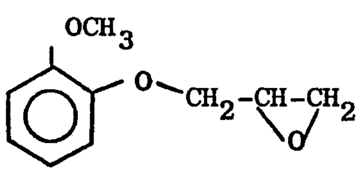 ; 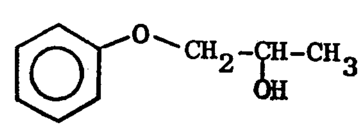 ;

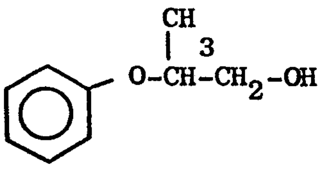 ; 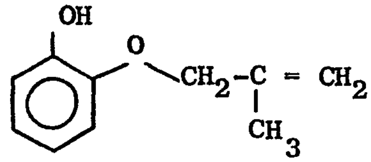 ;

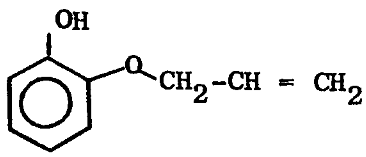 ; 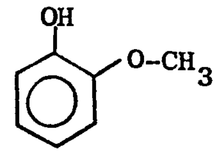 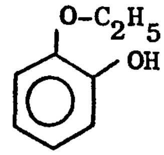

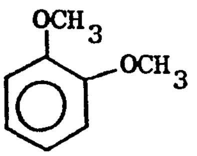 ; 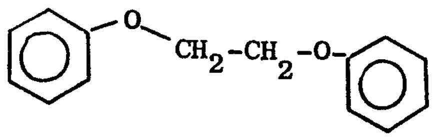 ;

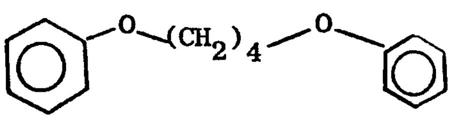

Ces composés sont utiles notamment comme intermédiaires de synthèse pour des produits phytosanitaires ou pharmaceutiques.

Les agents séquestrants utilisés dans le procédé selon l'invention peuvent être préparés de la façon suivante :

Ces composés peuvent être préparés par condensation d'un sel de formule :

$$R_5\ (O - \underset{\displaystyle CH}{\overset{R_4}{|}} - \underset{\displaystyle CH}{\overset{R_3}{|}})_n - O\text{-}M$$

où $R_3$, $R_4$, $R_5$ et n ont la signification précédente et où M représente un atome de métal alcalin choisi parmi le sodium, le potassium et le lithium, soit sur une amine de formule générale :

$$N\text{-}(\underset{\displaystyle CH}{\overset{R_1}{|}} - \underset{\displaystyle CH}{\overset{R_2}{|}} - X)_3$$

dans laquelle $R_1$ et $R_2$ ont la signification précédente et X représente le chlore ou le brome, soit sur le chlorhydrate ou le bromhydrate correspondant.

Le rapport molaire sel de métal alcalin/amine est compris entre environ 3 et environ 5.

L'opération de condensation est réalisée à une température comprise entre 100 et 150 °C pendant 1 à 15 h en présence d'un solvant qui peut être par exemple le chlorobenzène ou de préférence le monoalkyléther d'éthylène glycol de formule :

$$R_5\text{—}(O\text{—}CHR_4\text{—}CHR_3)_n\text{—}OH$$

On opère de préférence de telle sorte qu'on ait une solution contenant de 2 à 5 moles de sel de métal alcalin par litre de solvant.

Le mélange en fin de réaction contient principalement l'amine tertiaire de formule :

$$N\text{-}[\underset{\displaystyle CH}{\overset{R_1}{|}}\text{-}\underset{\displaystyle CH}{\overset{R_2}{|}}\text{-}O\text{-}(\underset{\displaystyle CH}{\overset{R_3}{|}}\text{-}\underset{\displaystyle CH}{\overset{R_4}{|}}\text{-}O)_n\text{-}R_5]_3$$

mais contient aussi en faible proportion de l'amine secondaire correspondant :

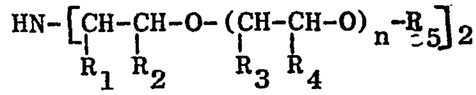

et des traces d'amines primaire :

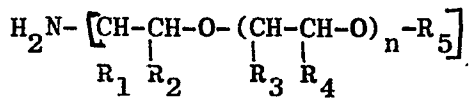

Les amines tertiaires, secondaires et primaires sont généralement respectivement dans le rapport 90 : 8 : 2 après distillation.

On peut utiliser dans le procédé selon l'invention directement le mélange ci-dessus obtenu après première distillation, c'est-à-dire contenant les trois types d'amines.

On préfère pour une meilleure mise en œuvre de l'invention effectuer une distillation plus poussée du mélange ci-dessus afin d'obtenir une amine tertiaire sensiblement pure.

D'autres caractéristiques et avantages apparaîtront à la lecture des exemples qui vont suivre. Il va de soi que ces exemples qui sont donnés uniquement à titre illustratif ne sauraient être considérés comme limitant la portée de la présente invention.

Exemple 1

Préparation de l'éther n-butylique du phénol, de formule :

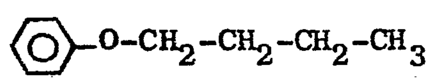

par réaction de phénate de sodium et de chlorure de n-butyle dans le chlorobenzène, en présence de tris(dioxa-3,6 heptyl)amine.

Dans le ballon tricol de 500 ml on introduit successivement 200 $cm^3$ de chlorobenzène anhydre, 11,6 g de phénate de sodium anhydre (1 mole), 12 g de chlorure de n-butyle (0,13 mole) et 1,6 g de tris(dioxa-3,6 heptyl)amine (0,005 mole).

Le milieu réactionnel est agité et chauffé à 80 °C. Le rendement de la réaction est de 78 % au bout de 29 h et de 98 % après 46 h.

Exemple comparatif

Dans les mêmes conditions opératoires, en l'absence de la tris(dioxa-3,6 heptyl)amine, on n'observe pas de formation de l'éther n-butylique du phénol.

Exemple 2

Préparation du phénoxy-3 propène-1, de formule :

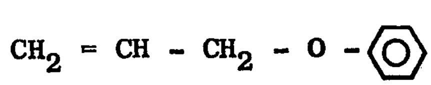

à partir de phénate de sodium et de chlorure d'allyle dans le chlorobenzène en présence de tris(dioxa-3,6 heptyl)amine.

Dans un ballon tricol de 500 ml équipé d'un agitateur mécanique, d'un thermomètre et d'un réfrigérant ascendant on introduit successivement 200 $cm^3$ de chlorobenzène anhydre, 11,6 g (0,1 mole) de phénate de sodium anhydre, 10 g de chlorure d'allyle (0,13 mole) et 1,6 g de tris(dioxa-3,6 heptyl)amine (0,005 mole).

Le milieu réactionnel est chauffé à 45 °C.

Après 6 h de réaction, le rendement est de 47 % et de 99 % après 23 h.

Exemple comparatif

En l'absence de la tris(dioxa-3,6 heptyl)amine, dans les mêmes conditions opératoires, on n'observe pas la formation de l'éther allylique.

## Exemple 3

Préparation de l'éther glycidique de l'orthocrésol, de formule :

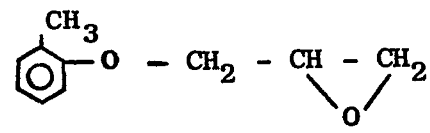

par réaction de l'orthocrésolate de sodium et de l'épichlorhydrine

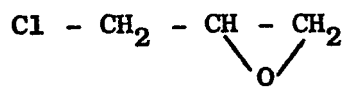

dans le tétrahydrofuranne en présence de tris(dioxa-3,6 octyl)amine.

Dans un ballon tricol de 500 ml équipé d'un agitateur mécanique, d'un thermomètre et d'un réfrigérant ascendant on introduit successivement 300 $cm^3$ de tétrahydrofuranne anhydre, 65 g d'o-crésolate de sodium (0,5 mole), 51 g d'épichlorhydrine fraîchement distillée (0,55 mole) et 9,1 g de tris(dioxa-3,6 octyl)amine (0,025 mole). Le milieu réactionnel est chauffé à 67 °C. Le taux de transformation est de 51 % après 5 h de réaction.

### Exemple comparatif

En l'absence de tris(dioxa-3,6 octyl)amine, dans les mêmes conditions opératoires, le taux de transformation est de 24 % après 5 h de réaction.

## Exemple 4

Préparation de l'éther glycidique du gaïacol, par réaction du gaïacolate de sodium et d'épichlorhydrine (l'épichlorhydrine jouant le rôle de solvant) en présence de tris(dioxa-3,6 octyl)amine.

Préparation de gaïacolate de sodium

Dans un réacteur tricol de 3 litres, muni d'un agitateur mécanique, d'un réfrigérant et d'une ampoule de coulée, on introduit :

— 341 g de gaïacol (2,75 moles),
— 300 g d'eau,
— 700 g de toluène.

A l'aide d'une ampoule de coulée, on ajoute lentement une solution aqueuse de soude à 33 % (300 g soit 2,5 moles de soude) en veillant à ce que la température soit comprise entre 45 °C et 55 °C. On agite pendant 2 heures puis on laisse décanter. La couche aqueuse est concentrée jusqu'à précipitation du gaïacolate de sodium. Après refroidissement, on filtre les cristaux de gaïacolate puis on les sèche par distillation azéotropique en présence de toluène.

Après filtration, le solide est séché à 80-90 °C sous 20 mmHg pendant 8 h. Le rendement est de 95 %.

Préparation de l'éther glycidique du gaïacol.

Dans un ballon tétracol de 2 litres équipé d'un agitateur mécanique, d'un réfrigérant ascendant muni d'un piège $CaCl_2$ d'un thermomètre, on introduit 740 g d'épichlorhydrine distillée et conservée sur tamis, 18,3 g de tris(dioxa-3,6 octyl)amine (0,05 mole). Le mélange est chauffé à 118 °C sous courant d'azote. Le gaïacolate de sodium est introduit en 10 fractions de 0,1 mole chacune à 15 mn d'intervalle après la dernière fraction ajoutée on maintient le chauffage pendant 15 mn supplémentaires.

On laisse ensuite refroidir le mélange puis on filtre le NaCl formé [60 g (théorie = 58 g)] ; l'épichlorhydrine est récupérée au moyen d'un évaporateur rotatif sous 5 mmHg (température du bain d'huile : 80 °C).

rut obtenu (205 g) est distillé : la fraction principale est composée de l'éther glycidique du gaïacol ($\theta$ = 96-109° sous 0,1 mmHg). La quantité de produit recueilli correspond à un rendement de 93 % par rapport au gaïacolate introduit.

On récupère une deuxième fraction distillant entre 130 °C et 160 °C sous 0,2 mmHg ; cette fraction est constituée par 14 g de tris(dioxa-3,6 octyl)amine (77 % de l'agent séquestrant engagé).

Exemple comparatif

Sans tris(dioxa-3,6 octyl)amine, le rendement de la réaction est de 68 %.

Exemple 5

Préparation de l'éther glycidique du gaïacol de formule :

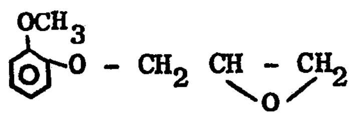

par réaction de gaïacolate de sodium, de formule :

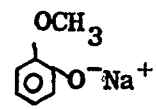

et d'épichlorhydrine dans l'acétonitrile en présence de tris(dioxa-3,6 octyl)amine.

Dans un ballon tricol de 500 ml équipé d'un réfrigérant ascendant, d'un thermomètre et d'un agitateur mécanique, on introduit successivement 300 $cm^3$ d'acétonitrile anhydre, 73 g de gaïacolate de sodium (0,5 mole) 51 g d'épichlorhydrine (0,55 mole) et 9,1 g de tris(dioxa-3,6 octyl)amine (0,025 mole).

Le mélange réactionnel est chauffé à reflux de l'acétonitrile sans agitation. Le taux de transformation est de 60 % après 5 h de réaction.

Exemple comparatif

Dans les mêmes conditions opératoires, en l'absence de la tris(dioxa-3,6 octyl)amine, le taux de transformation est de 25 % après 5 h de réaction.

Exemple 6

Préparation du monoéther, de formule :

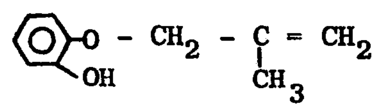

par réaction du pyrocatécholate de sodium, de formule :

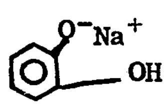

et du chlorure de méthallyle

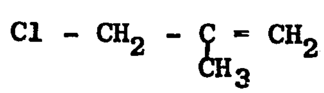

dans le toluène en présence de tris(dioxa-3,6 octyl)amine.

Dans un ballon tricol de 250 ml équipé d'un agitateur mécanique, d'un réfrigérant ascendant et d'un thermomètre on introduit successivement 70 $cm^3$ de toluène, 6,6 g de pyrocatécholate de sodium (0,05 mole), 9,1 g de chlorure de méthallyle distillé (0,1 mole) et 1,5 g de tris(dioxa-3,6 octyl)amine (0,005 mole). On fait passer un courant d'argon puis on chauffe le mélange réactionnel à 100 °C pendant 5 h. Le taux de transformation du pyrocatécholate de sodium est de 55 %. Le rendement en monoéther formé est de 92 %.

Exemple comparatif

Dans les mêmes conditions, en l'absence de la tris(dioxa-3,6 octyl)amine, le taux de transformation est inférieur à 5 %.

## Exemples 7 à 10

Préparation du monoéther, de formule :

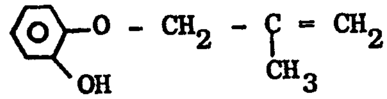

par réaction du pyrocatécholate de sodium et du chlorure de méthallyle dans divers solvants et en présence de tris(dioxa-3,6 octyl)amine.

On opère comme dans l'exemple 6, en remplaçant le toluène par le chlorobenzène (ex. 7 et 8), l'anisole (ex. 9) et l'acétonitrile (ex. 10). Les résultats sont donnés dans le tableau I.

TABLEAU I

| Ex. | Solvant | Durée | Taux de transformation | Sélectivité |
|---|---|---|---|---|
| 7 | chlorobenzène | 4 h 15 | 67 % | 95 % |
| 8 | chlorobenzène | 5 h 55 | 74 % | 93 % |
| 9 | anisole | 6 h 30 | 73,5 % | 94,5 % |
| 10 | acétonitrile | 10 h 30 | 67,5 % | 90 % |

## Exemples 11 et 12

On opère comme dans l'exemple 6 mais en remplaçant la tris(dioxa-3,6 octyl)amine par la tris(dioxa-3,6 heptyl) amine et en remplaçant le toluène par le chlorobenzène (ex. 11) et l'anisole (ex. 12).

Les résultats obtenus sont donnés dans le tableau II.

TABLEAU II

| Ex. | Solvant | Durée | Taux de transformation | Sélectivité |
|---|---|---|---|---|
| 11 | chlorobenzène | 2 h 30 | 39 % | 95 % |
| 12 | anisole | 3 h 30 | 66 % | 93 % |

## Exemple 13

Réaction du pyrocatécholate de sodium avec le bromure d'éthyle dans l'acétonitrile en présence de tris(dioxa-3,6 heptyl)amine.

Dans un ballon tricol de 500 ml équipé d'un agitateur mécanique, d'un thermomètre et d'un réfrigérant ascendant, on introduit successivement 200 ml d'acétonitrile, 13,2 g de pyrocatécholate de sodium (0,1 mole), 16,4 g de bromure d'éthyle (0,15 mole) et 1,6 g de tris(dioxa-3,6 heptyl)amine (0,005 mole). Le mélange est agité et chauffé à 40 °C pendant 8 h. Le taux de transformation du pyrocatécholate de sodium est de 70 %.

## Exemple comparatif

Dans les mêmes conditions opératoires, en l'absence de tris(dioxa-3,6 heptyl)amine, le taux de transformation de pyrocatécholate de sodium n'est que de 8 %.

## Exemple 14

Réaction du pyrocatécholate de sodium avec le bromure d'éthyle dans l'acétonitrile en présence de

tris(trioxa-3,6,9 décyl)amine.

Dans l'appareillage défini précédemment, on introduit 200 ml d'acétonitrile, 14,8 g de pyrocatécholate de potassium (0,1 mole) 16,4 g de bromure d'éthyle (0,15 mole) et 2,27 g de tris(trioxa-3,6,9 décyl)amine (0,005 mole). Le mélange est agité et chauffé à 40 °C pendant 8 h. Le taux de transformation du pyrocatécholate de potassium est de 87 %.

Exemple 15

Synthèse du (méthyl-2, chloro-4, phénoxy)-2 propionate de méthyle par réaction du méthyl-2 chloro-4 phénate de sodium avec le chloro-2 propionate de méthyle dans le toluène en présence de tris(dioxa-3,6 octyl)amine.

Dans un ballon tricol de 500 ml, équipé d'un agitateur mécanique, d'un thermomètre et d'un réfrigérant ascendant, on introduit successivement 250 $cm^3$ de toluène, 32,9 g de méthyl-2, chloro-4, phénate de sodium (0,2 mole), 24,5 g de chloro-2 propionate de méthyle (0,2 mole) et 1,8 g de tris(dioxa-3,6 octyl)amine (0,005 mole). Le mélange est chauffé à 50 °C pendant 7 h puis refroidi à 20 °C. On ajoute alors 100 $cm^3$ d'eau afin d'éliminer les sels formés et le chloro-crésolate de sodium non transformé. La phase organique est séchée sur silicagel puis le toluène est évaporé. Le (méthyl-2, chloro-4 phénoxy-2, propionate de méthyle est récupéré par distillation ($\theta$ = 91-93 °C sous 0,5 mmHg). Le rendement de la réaction est de 74 %.

Exemple comparatif

Dans les mêmes conditions opératoires, en l'absence de tris(dioxa-3,6 octyl)amine, le rendement de la réaction est de 12 %.

Exemple 16

Préparation de la tris(dioxa-3,6 octyl)amine.

a) Dans un ballon tricol d'un litre, muni d'un agitateur mécanique, d'un thermomètre, d'un réfrigérant, on introduit 450 g d'éthoxy-2 éthanol (5 moles). On ajoute 23 g de sodium (1 mole), en 3 h en maintenant la température de mélange à 40 °C.

b) Au mélange précédent, on ajoute 51,6 g de chlorhydrate de tris(chloro-2 éthyl)amine (soit 0,215 mole). On chauffe alors le mélange à reflux pendant 12 h puis on distille le solvant sous pression réduite. Le éthoxy-2 éthanolate de sodium en excès est neutralisé par addition de 12 $cm^3$ d'HCl aqueux (10 N).

Le chlorure de sodium formé est filtré et la solution est distillée. La tris(dioxa-3,6 octyl)amine distille entre 200 °C et 210 °C sous 1 mmHg. Le rendement est de 68 %.

Exemple 17

Préparation de la tris(dioxa-3,6 heptyl)amine.

a) Dans un ballon tricol d'un litre, muni d'un agitateur mécanique, d'un thermomètre, d'un réfrigérant, on introduit 380 g de méthoxy-2 éthanol (5 moles). On ajoute 23 g de sodium (1 mole) en 3 h en maintenant la température du mélange à 40 °C.

b) Au mélange précédent, on ajoute 51,6 g de chlorhydrate de tris(chloro-2 éthyl)amine (soit 0,215 mole). On chauffe alors le mélange à reflux (125 °C) pendant 12 h, puis on distille le solvant sous pression réduite. Le méthoxy-2 éthanolate de sodium en excès est neutralisé par addition de 11,6 $cm^3$ d'HCl aqueux (10 N). Le chlorure de sodium est filtré et la solution distillée.

Exemple 18

Préparation de la tris(trioxa-3,6,9 décyl)amine.

Dans un ballon tricol d'un litre équipé d'un agitateur mécanique, d'un condenseur et d'un thermomètre, on introduit 600 g d'éther monométhylique du diéthylèneglycol (dioxa-3,6 heptanol-1) soit 5 moles puis 23 g de sodium (1 mole) par petites fractions afin de former le dioxa-3,6 heptanolate de sodium.

Lorsque le sodium est totalement transformé, on ajoute alors 51,8 g de chlorhydrate de la tris(chloro-2 éthyl)amine (soit 0,215 mole). Le mélange est chauffé à 130 °C pendant 8 h sous agitation puis refroidi et l'excès d'alcoolate de sodium neutralisé par une solution aqueuse d'acide chlorhydrique à 10 %. Le dioxa-3,6 heptanol-1 est éliminé par distillation à 130 °C sous 20 mmHg. Le mélange obtenu est filtré afin d'éliminer le chlorure de sodium puis le produit est distillé. On obtient ainsi 83 g de tris(trioxa-3,6,9 décyl)amine qui distille à 189 °C sous 0,1 mmHg.

**Revendications**

1. Procédé de préparation d'éther arylique par réaction en milieu solvant organique d'un halogénure aliphatique avec un phénolate ou un naphtolate d'un métal alcalin ou alcalino-terreux ou d'ammonium, caractérisé en ce que la réaction a lieu en présence d'au moins un agent séquestrant de formule :

$$N-[CHR_1-CHR_2-O-(CHR_3-CHR_4-O)_n-R_5]_3 \qquad (I)$$

dans laquelle n est un nombre entier supérieur ou égal à 0 et inférieur ou égal à 10($0 \leq n \leq 10$), $R_1$, $R_2$, $R_3$ et $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical $-C_mH_{2m}-\emptyset$ ou $C_mH_{2m+1}-\emptyset-$, où m est compris entre 1 et 12.

2. Procédé selon la revendication 1, caractérisé en ce que dans la formule (I) $R_1$, $R_2$, $R_3$ et $R_4$ représentent un atome d'hydrogène ou un radical méthyle.

3. Procédé selon la revendication 1, caractérisé en ce que dans la formule (I) n est un nombre entier supérieur ou égal à 0 et inférieur ou égal à 6.

4. Procédé selon la revendication 1, caractérisé en ce que dans la formule (I) $R_5$ représente un radical alkyle ayant de 1 à 4 atomes de carbone.

5. Procédé selon les revendications 1-4, caractérisé en ce que dans la formule (I) $R_1$, $R_2$, $R_3$ et $R_4$ identiques ou différents représentent un atome d'hydrogène ou un radical méthyle, n est un nombre entier supérieur ou égal à 0 et inférieur ou égal à 6 et $R_5$ représente un radical alkyle ayant de 1 à 4 atomes de carbone.

6. Procédé selon la revendication 5, caractérisé en ce que l'agent séquestrant de formule (I) est la tris(dioxa-3,6 heptyl)amine de formule :

$$N-(CH_2-CH_2-O-CH_2-CH_2-O-CH_3)_3$$

7. Procédé selon la revendication 5, caractérisé en ce que l'agent séquestrant de formule (I) est la tris(trioxa-3,6,9 décyl)amine de formule :

$$N-(CH_2-CH_2-O-CH_2-CH_2-O-CH_2-CH_2-O-CH_3)_3$$

8. Procédé selon la revendication 5, caractérisé en ce que l'agent séquestrant de formule (I) est la tris(dioxa-3,6 octyl)amine de formule :

$$N-(CH_2-CH_2-O-CH_2-CH_2-O-C_2H_5)_3$$

9. Procédé selon l'une quelconque des revendications précédentes, caractérisé en ce qu'on opère en présence d'un solvant apolaire.

10. Procédé selon la revendication 9, caractérisé en ce qu'on opère en présence d'un solvant choisi parmi le groupe comprenant le benzène, le toluène, l'o-xylène, le m-xylène, le p-xylène, le monochlorobenzène, l'orthodichlorobenzène, le chloroforme, l'anisole, le diphényléther, l'acétonitrile, le dichloroéthane, le chlorobutane, le chlorure de benzyle, le nitrométhane, la diméthylformamide, la diméthylacétamide.

11. Procédé selon l'une quelconque des revendications 1 à 8, caractérisé en ce qu'on opère en présence d'un solvant constitué par l'halogénure aliphatique.

12. Procédé selon l'une quelconque des revendications précédentes, caractérisé en ce qu'on utilise l'agent séquestrant de formule I en quantité telle que le rapport molaire de l'agent séquestrant au phénolate ou au naphtolate est compris entre 0,5/100 et 15/100.

13. Procédé selon la revendication 12, caractérisé en ce que le rapport molaire de l'agent séquestrant au phénolate ou au naphtolate est compris entre 1/100 et 10/100.

14. Procédé selon l'une quelconque des revendications précédentes, caractérisé en ce que le rapport molaire de l'halogénure aliphatique au phénolate ou au naphtolate est compris entre 10 et 0,8.

15. Procédé selon l'une quelconque des revendications précédentes, caractérisé en ce qu'on opère à une température comprise entre − 25 °C e 150 °C.

16. Procédé de préparation selon l'une ou plusieurs des revendications précédentes d'un composé de formule :

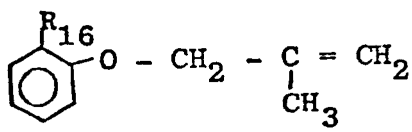

dans laquelle $R_{16}$ représente un radical choisi parmi le groupe comprenant les radicaux OH, alkoxy ayant

de 1 à 4 atomes de carbone, F, Cl, Br, $NO_2$, alcanoyle ayant de 1 à 4 atomes de carbone, alkyl CONH— et alkyl ayant de 1 à 4 atomes de carbone par réaction d'un sel alcalin du phénol de formule :

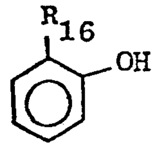

où $R_{16}$ a la signification précédente avec le chlorure de méthallyle.

17. Procédé de préparation selon l'une ou plusieurs des revendications précédentes du composé de formule :

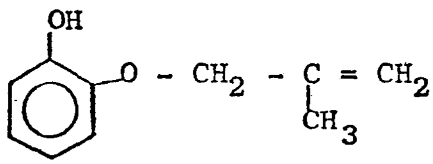

par réaction du pyrocatécholate de sodium avec le chlorure de méthallyle en milieu solvant organique, caractérisé en ce que l'on opère en présence de tris(dioxa-3,6 octyl)amine de formule :

$$N—(CH_2—CH_2—O—CH_2—CH_2—O—C_2H_5)_3$$

18. Procédé selon la revendication 17, caractérisé en ce que l'on opère en présence d'un solvant choisi parmi le groupe comprenant le toluène, le chlorobenzène, l'anisole, et l'acétonitrile.

19. Procédé de préparation selon l'une ou plusieurs des revendications précédentes du composé de formule :

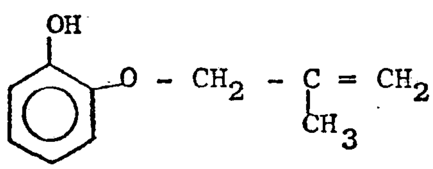

par réaction du pyrocatécholate de sodium avec le chlorure de méthallyle en milieu solvant organique, caractérisé en ce que l'on opère en présence de tris(dioxa-3,6 heptyl)amine de formule :

$$N—(CH_2—CH_2—O—CH_2—CH_2—O—CH_3)_3$$

20. Procédé selon la revendication 19, caractérisé en ce que l'on opère en présence d'un solvant choisi parmi le groupe comprenant le chlorobenzène et l'anisole.

**Claims**

1. Process for the preparation of an aryl ether by reacting, in an organic solvent medium, an aliphatic halide with an alkali metal phenolate or naphtholate, an alkaline earth metal phenolate or naphtholate or an ammonium phenolate or naphtholate, characterised in that the reaction takes place in the presence of at least one sequestering agent of the formula :

$$N—[CHR_1—CHR_2—O—(CHR_3—CHR_4—O)_n—R_5]_3 \qquad (I)$$

in which n is an integer which is greater than or equal to 0 and less than or equal to 10 ($0 \leq n \leq 10$), $R_1$, $R_2$, $R_3$ and $R_4$, which are identical or different, represent a hydrogen atom or an alkyl radical having from 1 to 4 carbon atoms and $R_5$ represents an alkyl or cycloalkyl radical having from 1 to 12 carbon atoms, a phenyl radical or a radical $—C_mH_{2m}—Ø$ or $C_mH_{2m+1}—Ø—$, in which m is between 1 and 12.

2. Process according to Claim 1, characterised in that, in the formula (I), $R_1$, $R_2$ and $R_4$ represent a hydrogen atom or a methyl radical.

3. Process according to Claim 1, characterised in that, in the formula (I), n is an integer which is greater than or equal to 0 and less than or equal to 6.

4. Process according to Claim 1, characterised in that, in the formula (I), $R_5$ represents an alkyl radical having from 1 to 4 carbon atoms.

5. Process according to Claims 1-4, characterised in that, in the formula (I), $R_1$, $R_2$, $R_3$ and $R_4$, which are identical or different, represent a hydrogen atom or a methyl radical, n is an integer which is greater than or equal to 0 less than or equal to 6 and $R_5$ represents an alkyl radical having from 1 to 4 carbon atoms.

6. Process according to Claim 5, characterised in that the sequestering agent of the formula (I) is tris(3,6-dioxaheptyl)-amine of the formula :

$$N—(CH_2—CH_2—O—CH_2—CH_2—O—CH_3)_3.$$

7. Process according to Claim 5, characterised in that the sequestering agent of the formula (I) is tris-(3,6,9-trioxadecyl)-amine of the formula :

$$N—(CH_2—CH_2—O—CH_2—CH_2—O—CH_2—CH_2—O—CH_3)_3.$$

8. Process according to Claim 5, characterised in that the sequestering agent of the formula (I) is tris(3,6-dioxaoctyl)-amine of the formula :

$$N—(CH_2—CH_2—O—CH_2—CH_2—O—C_2H_5)_3.$$

9. Process according to any one of the preceding claims, characterised in that the reaction is carried out in the presence of an apolar solvent.

10. Process according to Claim 9, characterised in that the reaction is carried out in the presence of a solvent chosen from amongst the group comprising benzene, toluene, o-xylene, m-xylene, p-xylene, monochlorobenzene, ortho-dichlorobenzene, chloroform, anisole, diphenyl ether, acetonitrile, dichloroethane, chlorobutane, benzyl chloride, nitromethane, dimethylformamide and dimethylacetamide.

11. Process according to any one of Claims 1 to 8, characterised in that the reaction is carried out in the presence of a solvent consisting of the aliphatic halide.

12. Process according to any one of the preceding claims, characterised in that the amount of sequestering agent of the formula I used is such that the molar ratio of the sequestering agent to the phenolate or naphtholate is between 0,5/100 and 15/100.

13. Process according to Claim 12, characterised in that the molar ratio of the sequestering agent to the phenolate or naphtholate is between 1/100 and 10/100.

14. Process according to any one of the preceding claims, characterised in that the molar ratio of the aliphatic halide to the phenolate or naphtholate is between 10 and 0.8.

15. Process according to any one of the preceding claims, characterised in that the reaction is carried out at a temperature between − 25 °C and 150 °C.

16. Process according to one or more of the preceding claims, for the preparation of a compound of the formula :

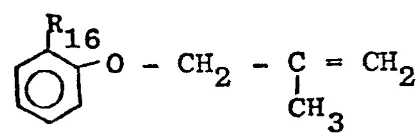

in which $R_{16}$ represents a radical chosen from amongst the group comprising the OH radical, alkoxy radicals having from 1 to 4 carbon atoms, F, Cl, Br and $NO_2$ radicals, alkanoyl radicals having from 1 to 4 carbon atoms, and alkyl CONH— and alkyl radicals having from 1 to 4 carbon atoms, by reacting an alkali metal salt of the phenol of the formula :

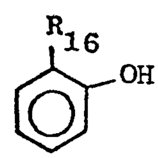

in which $R_{16}$ has the above meaning, with methallyl chloride.

17. Process according to one or more of the preceding claims, for the preparation of the compound of the formula :

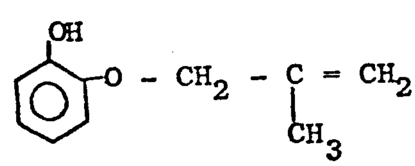

by reacting sodium pyrocatecholate with methallyl chloride in an organic solvent medium, characterised in that the reaction is carried out in the presence of tris-(3,6-dioxaoctyl)-amine of the formula :

$$N—(CH_2—CH_2—O—CH_2—CH_2—O—C_2H_5)_3.$$

18. Process according to Claim 17, characterised in that the reaction is carried out in the presence of a solvent chosen from amongst the group comprising toluene, chlorobenzene, anisole and acetonitrile.

19. Process according to one or more of the preceding claims, for the preparation of the compound of the formula :

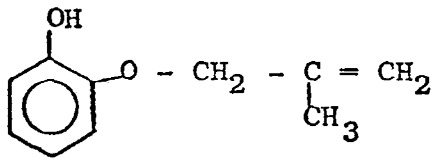

by reacting sodium pyrocatecholate with methallyl chloride in an organic solvent medium, characterised in that the reaction is carried out in the presence of tris-(3,6-dioxaheptyl)-amine of the formula :

$$N—(CH_2—CH_2—O—CH_2—CH_2—O—CH_3)_3.$$

20. Process according to Claim 19, characterised in that the reaction is carried out in the presence of a solvent chosen from amongst the group comprising chlorobenzene and anisole.

**Ansprüche**

1. Verfahren zur Herstellung von Aryläther durch Reaktion eines aliphatischen Halogenides mit einem Phenolat oder Naphtolat von einem Alkali- oder Erdalkalimetall oder Ammonium, in einem organischen Lösungsmittel, dadurch gekennzeichnet, daß die Reaktion in Gegenwart mindestens eines Sequestrierungsmittels der Formel :

$$N\text{-}[CHR_1—CHR_2—O—(CHR_3—CHR_4—O)_n—R_5]_3 \quad (I)$$

durchgeführt wird, in der n eine ganze Zahl größer oder gleich 0 und kleiner oder gleich 10 ($0 \leq n \leq 10$) ist, $R_1$, $R_2$, $R_3$ und $R_4$ gleich oder unterschiedlich ein Wasserstoffatom oder einen Alkylrest mit 1 bis 4 Kohlenstoffatomen darstellen und $R_5$ einen Alkyl- oder Cycloalkylrest mit 1 bis 12 Kohlenstoffatomen, einen Phenylrest oder einen $—C_mH_{2m}—Ø$ oder $C_mH_{2m+1}—Ø—$Rest bedeuten, in dem m zwischen 1 und 12 liegt.

2. Verfahren nach Anspruch 1, dadurch gekennzeichnet, daß in der Formel (I) $R_1$, $R_2$, $R_3$ und $R_4$ ein Wasserstoffatom oder einen Methylrest bedeuten.

3. Verfahren nach Anspruch 1, dadurch gekennzeichnet, daß in der Formel (I) n eine ganze Zahl größer oder gleich 0 und kleiner oder gleich 6 ist.

4. Verfahren nach Anspruch 1, dadurch gekennzeichnet, daß in der Formel (I) $R_5$ einen Alkylrest mit 1 bis 4 Kohlenstoffatomen bedeutet.

5. Verfahren nach den Ansprüchen 1 bis 4, dadurch gekennzeichnet, daß in der Formel (I) $R_1$, $R_2$, $R_3$. und $R_4$ gleich oder unterschiedlich ein Wasserstoffatom oder einen Methylrest darstellen, n eine ganze Zahl größer oder gleich 0 und kleiner oder gleich 6 ist und $R_5$ einen Alkylrest mit 1 bis 4 Kohlenstoffatomen bedeutet.

6. Verfahren nach Anspruch 5, dadurch gekennzeichnet, daß das Sequestrierungsmittel der Formel (I) das Tris-(dioxa-3,6-heptyl)amin der Formel :

$$N—(CH_2—CH_2—O—CH_2—CH_2—O—CH_3)_3$$

ist.

7. Verfahren nach Anspruch 5, dadurch gekennzeichnet, daß das Sequestrierungsmittel der Formel (I) das Tris-(trioxa-3,6,9-decyl)amin der Formel

$$N—(CH_2—CH_2—O—CH_2—CH_2—O—CH_2—CH_2—O—CH_3)_3$$

ist.

8. Verfahren nach Anspruch 5, dadurch gekennzeichnet, daß das Sequestrierungsmittel der Formel (I) das Tris(dioxa-3,6-octyl)amin der Formel :

$$N-(CH_2-CH_2-O-CH_2-CH_2-O-C_2H_5)_3$$

ist.

9. Verfahren nach irgendeinem der vorstehenden Ansprüche, dadurch gekennzeichnet, daß man in Gegenwart eines unpolaren Lösungsmittels arbeitet.

10. Verfahren nach Anspruch 9, dadurch gekennzeichnet, daß man in Gegenwart eines Lösungsmittels arbeitet, das aus der Benzol, Toluol, o-Xylol, m-Xylol, p-Xylol, Monochlorbenzol, Orthodichlorbenzol, Chloroform, Anisol, Diphenyläther, Acetonitril, Dichloräthan, Chlorbutan, Benzylchlorid, Nitromethan, Dimethylformamid und Dimethylacetamid umfassenden Gruppe ausgewählt ist.

11. Verfahren nach irgendeinem der Ansprüche 1 bis 8, dadurch gekennzeichnet, daß man in Gegenwart eines Lösungsmittels arbeitet, das aus dem aliphatischen Halogenid besteht.

12. Verfahren nach irgendeinem der vorstehenden Ansprüche, dadurch gekennzeichnet, daß man das Sequestrierungsmittel der Formel (I) in einer Menge verwendet, daß das Molverhältnis des Sequestrierungsmittels zum Phenolat oder Naphtolat zwischen 0,5/100 und 15/100 liegt.

13. Verfahren nach Anspruch 12, dadurch gekennzeichnet, daß das Molverhältnis des Sequestrierungsmittels zum Phenolat oder Naphtolat zwischen 1/100 und 10/100 liegt.

14. Verfahren nach irgendeinem der vorstehenden Ansprüche, dadurch gekennzeichnet, daß das Molverhältnis des aliphatischen Halogenides zum Phenolat oder Naphtolat zwischen 10 und 0,8 liegt.

15. Verfahren nach irgendeinem der vorstehenden Ansprüche, dadurch gekennzeichnet, daß man bei einer Temperatur zwischen − 25 °C und 150 °C arbeitet.

16. Verfahren nach einem oder mehreren der vorstehenden Ansprüche zur Herstellung einer Verbindung der Formel

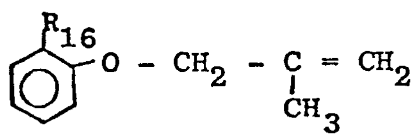

in der $R_{16}$ einen Rest bedeutet, der aus der Gruppe der Reste OH, Alkoxy mit 1 bis 4 Kohlenstoffatomen, F, Cl, Br, $NO_2$, Alcanoyl mit 1 bis 4 Kohlenstoffatomen, Alkyl-CONH und Alkyl mit 1 bis 4 Kohlenstoffatomen ausgewählt ist, durch Reaktion eines alkalischen Salzes eines Phenols der Formel

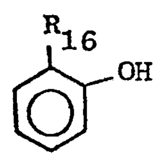

in der $R_{16}$ die vorstehende Bedeutung hat, mit Methallylchlorid.

17. Verfahren nach einem oder mehreren der vorstehenden Ansprüche zur Herstellung der Verbindung der Formel :

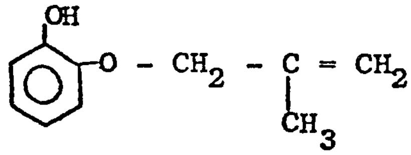

durch Reaktion des Natriumbrenzkatechins mit Methallylchlorid in einem organischen Lösungsmittel, dadurch gekennzeichnet, daß man in Gegenwart von Tris-(dioxa-3,6-octyl)amin der Formel

$$N-(CH_2-CH_2-O-CH_2-CH_2-O-C_2H_5)_3$$

arbeitet.

18. Verfahren nach Anspruch 17, dadurch gekennzeichnet, daß man in Gegenwart eines Lösungsmittels arbeitet, das aus der Toluol, Chlorbenzol, Anisol und Acetonitril umfassenden Gruppe ausgewählt ist.

19. Verfahren nach einem oder mehreren der vorstehenden Ansprüche zur Herstellung der Verbindung der Formel :

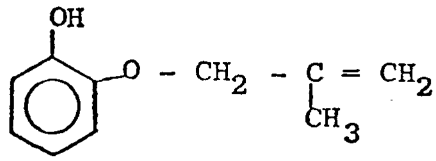

durch Reaktion des Natriumbrenzkatechins mit Methallylchlorid in einem organischen Lösungsmittel dadurch gekennzeichnet, daß man in Gegenwart von Tris(dioxa-3,6-heptyl)amin der Formel :

$$N—(CH_2—CH_2—O—CH_2—CH_2—O—CH_3)_3$$

arbeitet.

20. Verfahren nach Anspruch 19, dadurch gekennzeichnet, daß man in Gegenwart eines Lösungsmittels arbeitet, das aus der Chlorbenzol und Anisol umfassenden Gruppe ausgewählt wurde.